# EUROPEAN PATENT APPLICATION

(11) **EP 4 707 774 A1**
(43) Date of publication of application: **11.03.2026**
(21) Application number: 24845842.4
(22) Date of filing: 28.06.2024
(51) Int. Cl.: G01N 15/02, G01N 1/22, G01N 1/24, G01N 33/00, G01N 15/00

(54) **FOREIGN SUBSTANCE DETECTION DEVICE AND OPERATION METHOD THEREOF**

(30) Priority: 21.07.2023 KR 20230095446
(71) Applicant: LG Energy Solution, Ltd., Seoul 07335 (KR)
(72) Inventor: KIM, Do Yul, Daejeon 34122 (KR); BAE, Hye Yun, Daejeon 34122 (KR); KWON, Young Woo, Daejeon 34122 (KR)
(74) Representative: Plasseraud IP
(86) International application number: PCT/KR2024/009113
(87) International publication number: WO 2025/023538

(57) **Abstract**

A foreign substance detection device according to an embodiment disclosed in t he present document may include a suction part configured to suck outside air at a target point, a sensor configured to detect foreign substances contained in air sucked from the suction part, and a controller configured to adjust a flow rate of the air sucked from the suction part and determine the amount of foreign substances at the target point on the b asis of the foreign substances detected by the sensor.

## Description

### [Technical Field]

### CROSS-REFERENCE TO RELATED APPLICATION

This application claims priority to and the benefit of Korean Patent Application No. 10-2023-0095446 filed in the Korean Intellectual Property Office on July 21, 2023, the entire contents of which are incorporated herein by reference.

### TECHNICAL FIELD

Embodiments disclosed in the present document relate to a foreign substance detection device and a method of operating the same.

### [Background Art]

In case that foreign substances are inadvertently contained in particular products during a process of manufacturing the products, the produced products may be defective. In particular, in the case of precision products such as battery cells, the introduction of foreign substances during the manufacturing process may cause fatal defects.

Therefore, it is essential to determine a state of air by monitoring an airflow in a space, in which a product manufacturing process is performed, by using a fine dust sensor or the like during the product manufacturing process.

### [Disclosure]

### [Technical Problem]

However, there is a problem in that a general fine dust sensor or a device for detecting foreign substances merely monitors an approximate airflow in a space in which a manufacturing process is performed. In addition, there is a problem in that points, which may be measured by general devices, are limited because of a restrictive shape of an inlet. Therefore, there is a problem in that a particular point cannot be concentratedly monitored during the manufacturing process.

### [Technical Solution]

According to the embodiment disclosed in the present document, a foreign substance detection device may include: a suction part configured to suck outside air at a target point; a sensor configured to detect foreign substances contained in air sucked from the suction part; a flowmeter configured to measure a flow rate of the air sucked from the suction part; a pump configured to generate the flow rate; and a controller configured to adjust a duty of the pump on the basis of the flow rate measured by the flowmeter and determine the amount of foreign substances at the target point on the basis of the foreign substances detected by the sensor.

According to the embodiment, the controller may set a target flow rate and adjust the duty of the pump on the basis of a result of comparing the flow rate measured by the flowmeter with the target flow rate.

According to the embodiment, the controller may decrease the duty by a preset value when the flow rate measured by the flowmeter is higher than a preset ratio range of the target flow rate, and the controller may increase the duty by the preset value when the flow rate measured by the flowmeter is lower than the preset ratio range of the target flow rate.

According to the embodiment, the controller may determine whether the pump is abnormal and whether a filter configured to remove the foreign substances is abnormal on the basis of the duty of the pump and the flow rate measured by the flowmeter.

According to the embodiment, the controller may determine that the pump is abnormal when the flow rate is measured by the flowmeter in a state in which the duty of the pump 0% or when the flow rate measured by the flowmeter is not changed in a state in which the duty is changed.

According to the embodiment, the controller may determine that the filter is abnormal when the flow rate measured by the flowmeter is lower than the target flow rate in a state in which the duty of the pump is 100%.

According to the embodiment, an angle and a length of the suction part may be changed.

According to the embodiment disclosed in the present document, a method of operating a foreign substance detection device may include: sucking outside air at a target point; detecting foreign substances contained in the sucked air; measuring a flow rate of the sucked air; adjusting a duty of a pump, which is configured to generate the flow rate, on the basis of the measured flow rate; and determining the amount of foreign substances at the target point on the basis of the detected foreign substances.

According to the embodiment, the adjusting of the duty of the pump may include: setting a target flow rate; comparing the measured flow rate with the target flow rate; and adjusting the duty of the pump on the basis of a result of comparing the measured flow rate with the target flow rate.

According to the embodiment, the method may further include: determining whether the pump is abnormal and whether a filter configured to remove the foreign substances is abnormal on the basis of the duty of the pump and the measured flow rate.

### [Advantageous Effects]

According to the foreign substance detection device and the method of operating the same according to the embodiments disclosed in the present document, the length and the angle of the suction part may be changed, such that the amount of foreign substances at the desired target point may be determined.

In addition, according to the foreign substance detection device and the method of operating the same according to the embodiments disclosed in the present document, it is possible to determine the amount of foreign substances and simultaneously determine a defect of the component of the foreign substance detection device and/or whether the component needs to be replaced.

In addition, various effects that can be directly or indirectly identified through the present document may be provided.

### [Description of Drawings]

FIG. 1 is a block diagram illustrating a configuration of a foreign substance detection device according to the embodiment disclosed in the present document.
FIG. 2 is a view illustrating an example of a structure of the foreign substance detection device according to the embodiment disclosed in the present document.
FIG. 3 is a flowchart for explaining a method of operating the foreign substance detection device according to the embodiment disclosed in the present document.
FIG. 4 is a flowchart for explaining a method of adjusting a flow rate of air sucked by the foreign substance detection device according to the embodiment disclosed in the present document.
FIG. 5 is a flowchart illustrating a specific operating process of the foreign substance detection device according to the embodiment disclosed in the present document.

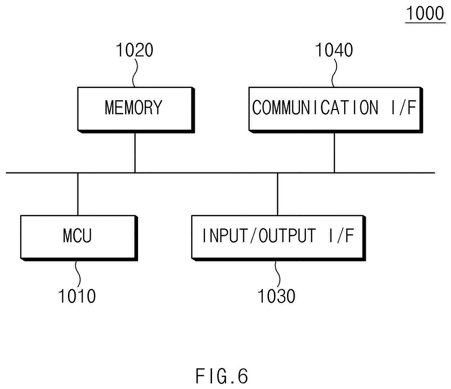

FIG. 6 is a block diagram illustrating a hardware configuration of a computing system for performing a method of operating a battery management device according to the embodiment disclosed in the present document.

### [Mode for Invention]

Hereinafter, various embodiments of the present disclosure disclosed herein will be described with reference to the accompanying drawings. However, the description of the embodiments is not intended to limit the present disclosure to the particular embodiments, but it should be understood that the present disclosure is to cover all modifications, equivalents, and/or alternatives of the embodiments of the present disclosure.

In the present document, a singular form of a noun corresponding to an item may include one or more of the items, unless the relevant context clearly indicates otherwise. In the present document, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). In case that an element (e.g., a first element) is referred to, with or without the term "operatively" or "communicatively," as "coupled with," "coupled to," "connected with," or "connected to" another element (e.g., a second element), it means that the element may be coupled with the other element directly (e.g., wiredly), wirelessly, or via a third element.

Each constituent element (e.g., module or program), among the constituent elements described in the present document, may include a single entity or a plurality of entities. According to various embodiments, one or more constituent elements, among the corresponding constituent elements, or operations may be omitted, or one or more other constituent elements or operations may be added. Alternatively or additionally, a plurality of components (e.g., modules or programs) may be integrated into a single component. In such a case, the integrated component may perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or a plurality of operations may be executed in a different order or omitted, or one or more other operations may be added.

The term "module" or "part" used in the present document may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to the embodiment, the module may be implemented in a form of an application-specific integrated circuit (ASIC).

Various embodiments in the present document may be implemented as software (e.g., programs or applications) including one or more instructions are stored in a storage medium (e.g., a memory) readable by a machine. For example, a processor of the machine may invoke at least one of the one or more instructions stored in the storage medium, and execute the at least one of the one or more instructions. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

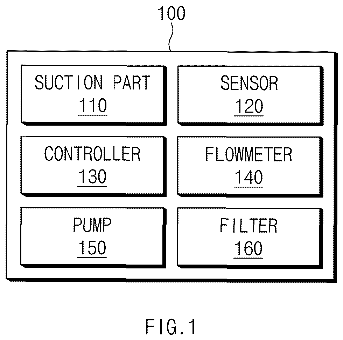

FIG. 1 is a block diagram illustrating a configuration of a foreign substance detection device according to the embodiment disclosed in the present document.

With reference to FIG. 1, a foreign substance detection device 100 may include a suction part 110, a sensor 120, a controller 130, a flowmeter 140, and a pump 150. The foreign substance detection device 100 may further include a filter 160.

The foreign substance detection device 100 may be used to determine the amount of foreign substances in a space, in which a manufacturing process is performed, during a manufacturing process of equipment during which a defect may occur because of introduction of foreign substances. For example, the foreign substance detection device 100 may be used in a space in which battery cells are manufactured. For example, the foreign substance detection device 100 may detect foreign substances that may be produced in a winding apparatus during a roll-to-roll process of manufacturing cylindrical cells.

The suction part 110 may suck outside air at a target point. The target point may be one region of the space in which the manufacturing process of the equipment is performed. For example, in case that the foreign substance detection device 100 is used to determine the amount of foreign substances during the battery cell manufacturing process, the target point may be a region adjacent to the process equipment, a region in which an operator's hand, face, or the like is positioned, a region in which a battery cell is positioned, or the like. In particular, the target point may be a point at which the likelihood of introduction of foreign substances into the product during the manufacturing process is relatively high.

At the target point, the suction part 110 may suck outside air into the foreign substance detection device 100. In this case, in case that foreign substances are present in the air in the vicinity of the target point, foreign substances, together with the air, may be introduced into the foreign substance detection device 100.

According to the embodiment, an angle and a length of the suction part 110 may be changed. That is, the suction part 110 may have a flexible shape. A user of the foreign substance detection device 100 may change the angle and the length of the suction part 110 in accordance with the position of the target point at which foreign substances are intended to be detected. The foreign substance detection device 100 uses the suction part 110 with a flexible shape. In general, devices with fixed inlets may detect foreign substances even at a position at which foreign substances cannot be detected. For example, the foreign substance detection device 100 may insert the suction part 110 into a gap in the process equipment and detect foreign substances in the process equipment.

According to the embodiment, the suction part 110 may have various shapes depending on the size, structure, purpose, and the like of a target in which foreign substances are to be detected. Because general devices for detecting foreign substances use inlets with fixed shapes, shapes of the inlets are restricted even though the inlets may be replaced and used. However, as described below, the foreign substance detection device 100 may adjust a flow rate of air sucked, such that the shape and the size of the suction part 110 may be comparatively freely configured.

The sensor 120 may detect foreign substances contained in the air sucked from the suction part. For example, the sensor 120 may detect whether foreign substances are present in the sucked air. In case that foreign substances are present, the sensor 120 may detect the amount of foreign substances (e.g., the number of foreign substances) and a concentration. For example, the sensor 120 may be an optical particle counter (OPC) capable of counting the number of foreign substances.

According to the embodiment, the sensor 120 may autonomously have an abnormality alarm function. The sensor 120 may transfer foreign substance detection information and abnormality information to the controller 130.

The controller 130 may adjust the flow rate of the air sucked from the suction part 110. The controller 130 may adjust the flow rate of the air sucked from the suction part 110 by adjusting a duty of the pump 150 on the basis of a flow rate measured by the flowmeter 140. In addition, the controller 130 may additionally adjust the flow rate of the air sucked from the suction part 110 by adjusting an opening degree of the suction part 110 and an angle defined with respect to the target point.

In particular, in case that the suction part 110 has a flexible shape, the flow rate is changed because a length and an angle of the suction part 110 are changed (the suction part 110 is bent) as the target point is changed when the foreign substance detection device 100 monitors the amount of foreign substances in the space. In this case, it is necessary to compensate for a change in flow rate caused by changes in the length and the angle of the suction part 110 in order to determine the amount of foreign substances under a condition in which the foreign substance detection device 100 remains the same. Therefore, the controller 130 may consistently adjust the flow rate of the air sucked from the suction part 110.

The controller 130 may determine the amount of foreign substances at the target point on the basis of the foreign substances detected by the sensor 120. For example, the controller 130 may determine the amount of foreign substances on the basis of the number of foreign substances, the concentration of foreign substances, and the like detected by the sensor 120. Further, the controller 130 may determine the suitability of the manufacturing process on the basis of the amount of foreign substances at the target point. For example, in case that the amount of foreign substances at the target point exceeds a preset level, the controller 130 may determine that the manufacturing process is insufficient, and ventilation and/or cleaning is required.

The foreign substance detection device 100 detects foreign substances by means of the sensor 120 and determines the amount of foreign substances at the target point on the basis of the detected foreign substances. Therefore, in order to improve detection reliability of the sensor 120 and determination accuracy of the controller 130, the foreign substance detection device 100 needs to operate under a constant flow rate condition. Therefore, the foreign substance detection device 100 may maintain the constant flow rate condition by adjusting the flow rate of the air sucked from the suction part 110.

The flowmeter 140 may measure the flow rate of the air sucked from the suction part 110. For example, in the foreign substance detection device 100, the air sucked from the suction part 110 may flow through a single pipe, and the flowmeter 140 may measure the flow rate of the air flowing through the corresponding pipe. The flowmeter 140 may transmit a flow rate measurement result to the controller 130.

The pump 150 may generate the flow rate of the air sucked from the suction part 110. In this case, the configuration in which the pump 150 generates the flow rate of the air may mean that the air may be introduced into the foreign substance detection device 100 from the suction part 110 by a pumping operation of the pump 150.

According to the embodiment, the controller 130 may adjust the duty of the pump 150 on the basis of the flow rate measured by the flowmeter 140. In this case, the configuration in which the controller 130 adjusts the duty of the pump 150 may include a configuration in which a pumping cycle of the pump 150 is adjusted. For example, the controller 130 may increase the duty of the pump 150 when the flow rate measured by the flowmeter 140 cannot reach a reference flow rate. In another example, in case that the flow rate measured by the flowmeter 140 consistently decreases, the controller 130 may increase the duty of the pump 150 to compensate for the decreased flow rate.

According to the embodiment, the controller 130 may set a target flow rate. The configuration in which the controller 130 sets the target flow rate may include a configuration in which information on the target flow rate is received from the user of the foreign substance detection device 100. The target flow rate may be set on the basis of a capacity of the inside of the foreign substance detection device 100, a cross-sectional area of the suction part 110, a rated flow rate of the sensor 120, and the like. For example, the controller 130 may set the target flow rate to the rated flow rate of the sensor 120. In this case, the foreign substance detection reliability of the sensor 120 may be improved.

According to the embodiment, the controller 130 may adjust the duty of the pump 150 on the basis of a result of comparing the flow rate, which is measured by the flowmeter 140, with the target flow rate. For example, the controller 130 may adjust the duty of the pump on the basis of a large/small relationship between the flow rate of the flowmeter 140 and the target flow rate. In another example, the controller 130 may adjust the duty of the pump 150 in proportion to a difference between the flow rate of the flowmeter 140 and the target flow rate.

The controller 130 may maintain the duty of the pump 150 when the flow rate measured by the flowmeter 140 is within a preset ratio range of the target flow rate. For example, the preset ratio range may be set on the basis of a degree to which the target flow rate may be within an error range in consideration of a measurement error of the flowmeter 140 or the like. In another example, the preset ratio range may be set on the basis of a minimum limit value of the amount of change in flow rate in accordance with the adjustment of the duty of the pump 150. For example, in case that the amount of change in flow rate is x% when the duty of the pump 150 is adjusted to a minimum unit, the preset ratio range may be set on the basis of x% (e.g., (100-x)% to (100+x)%). For example, the preset ratio range may be set to 95% to 105%.

According to the embodiment, the controller 130 may decrease the duty of the pump 150 by a preset value in case that the flow rate measured by the flowmeter 140 is higher than the preset ratio range of the target flow rate. On the contrary, the controller 130 may increase the duty of the pump 150 by a preset value in case that the flow rate measured by the flowmeter 140 is lower than the preset ratio range of the target flow rate.

The preset value may be determined in advance experimentally and statistically, and the value may vary depending on the states of the components of the foreign substance detection device 100. For example, the preset value may be set to 5%.

According to the embodiment, the controller 130 may determine whether the filter 160 and the pump 150 are abnormal on the basis of the duty of the pump 150 and the flow rate measured by the flowmeter 140. The filter 160 may remove foreign substances contained in the air sucked from the suction part 110. For example, the filter 160 may be an adsorption filter. In this case, foreign substances may be adsorbed to the filter 160 and removed. However, the type of filter 160 is not limited thereto.

According to the embodiment, the controller 130 may determine that the pump 150 is abnormal in case that the flow rate is measured by the flowmeter 140 in the state in which the duty of the pump 150 is 0%. In the state in which the duty of the pump 150 is 0%, the pump 150 does not perform the pumping operation, and the air need not be sucked through the suction part 110. The controller 130 may determine that the pump 150 is abnormal when the flow rate is measured by the flowmeter 140 in the state in which the duty of the pump 150 is 0%.

According to the embodiment, the controller 130 may determine that the pump 150 is abnormal in case that the flow rate measured by the flowmeter 140 is not changed in the state in which the duty of the pump 150 is changed. In case that the duty of the pump 150 is changed, the flow rate of the air sucked by the suction part 110 needs to be changed by a change in pumping cycle or the like. Therefore, the controller 130 may determine that the pump 150 is abnormal when the flow rate of the flowmeter 140 is not changed even though the duty of the pump 150 is changed.

According to the embodiment, it may be determined that the filter 160 is abnormal in case that the flow rate measured by the flowmeter 140 is lower than the target flow rate in the state in which the duty of the pump 150 is 100%. In the state in which the duty of the pump 150 is 100%, the flow rate of the air sucked from the suction part 110 may be a maximum flow rate. When the flow rate of the flowmeter 140 is lower than the target flow rate even though the flow rate of the sucked air is the maximum flow rate, it may be determined that the flow of air is hindered because foreign substances are adsorbed to the filter 160. Therefore, the controller 130 may determine that the filter 160 is abnormal in case that the flow rate of the flowmeter 140 is lower than the target flow rate in the state in which the duty of the pump 150 is 100%. In this case, the controller 130 may determine that the filter 160 needs to be replaced.

In case that the foreign substance detection device 100 determines that it is not appropriate to perform the process because the amount of foreign substances at the target point exceeds a reference degree or determines that at least any one of the sensor 120, the pump 150, and the filter 160 is abnormal, the foreign substance detection device 100 may provide determination information to the user. For example, the foreign substance detection device 100 may provide the determination information to the user through a display part (not illustrated) or a notification part (not illustrated).

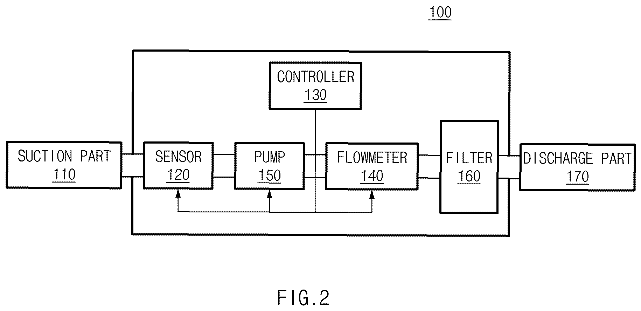

FIG. 2 is a view illustrating an example of a structure of the foreign substance detection device according to the embodiment disclosed in the present document.

With reference to FIG. 2, the foreign substance detection device 100 may suck the air through the suction part 110, detect foreign substances, remove the foreign substances, and discharge the air through a discharge part 170.

For example, the suction part 110 may protrude from a portion of the foreign substance detection device 100. The suction part 110 may suck air present outside the foreign substance detection device 100 at the target point.

The air sucked from the suction part 110 may move through a pipe in the foreign substance detection device 100. For example, the pipe in the foreign substance detection device 100 may connect the suction part 110 and the discharge part 170. The sensor 120, the pump 130, the flowmeter 140, and the filter 160 may be disposed in the pipe. The arrangement order of the sensor 120, the pump 130, and the flowmeter 140 illustrated in FIG. 2 is provided for illustrative purposes only, and the present disclosure is not limited thereto.

The sensor 120 may detect foreign substances contained in the air flowing through the pipe. For example, the sensor 120 may sense the number of foreign substances, the concentration of foreign substances, and the like.

For example, the pump 150 may generate the flow rate of the air sucked from the suction part 110 by performing the pumping operation.

The flowmeter 140 may measure the flow rate of the air sucked from the suction part 110.

The filter 160 may remove foreign substances contained in the sucked air. For example, the filter 160 may be an adsorption filter. In this case, foreign substances may be adsorbed to the filter 160 and removed.

The air, from which foreign substances are removed by the filter 160, may be discharged to the outside of the foreign substance detection device 100 through the discharge part 170.

The controller 130 may receive signals and/or information from the sensor 120 and the flowmeter 140. For example, the controller 130 may receive foreign substance detection information and abnormality information from the sensor 120 and receive measured flow rate information from the flowmeter 140. In addition, the controller 130 may set target information and adjust the duty of the pump 150 on the basis of the target information and the measured flow rate information.

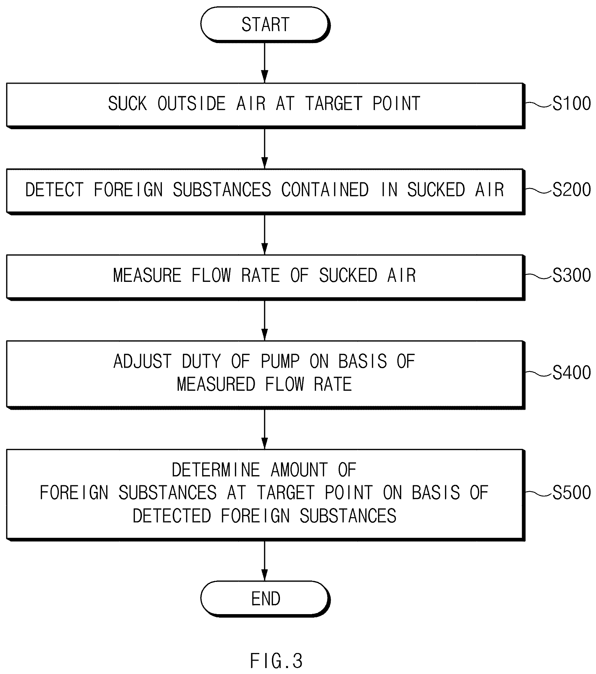

FIG. 3 is a flowchart for explaining a method of operating the foreign substance detection device according to the embodiment disclosed in the present document.

With reference to FIG. 3, the method of operating the foreign substance detection device may include step S100 of sucking outside air at the target point, step S200 of detecting foreign substances contained in the sucked air, step S300 of measuring a flow rate of the sucked air, step S400 of adjusting a duty of the pump on the basis of the measured flow rate, and step S500 of determining the amount of foreign substances at the target point on the basis of the detected foreign substances.

In step S100, the suction part 110 may suck outside air at the target point. The target point may be one region of the space in which the manufacturing process of the equipment is performed. In the embodiment, the angle and the length of the suction part 110 may be changed, and various points may be selected as the target points.

In step S200, the sensor 120 may detect foreign substances contained in the sucked air.

In step S300, the flowmeter 140 may measure the flow rate of the sucked air.

In step S400, the controller 130 may adjust the duty of the pump 150 on the basis of the measured flow rate.

In step S500, the controller 130 may determine the amount of foreign substances at the target point on the basis of the detected foreign substances. The controller 130 may provide the user with the result of determining the amount of foreign substances.

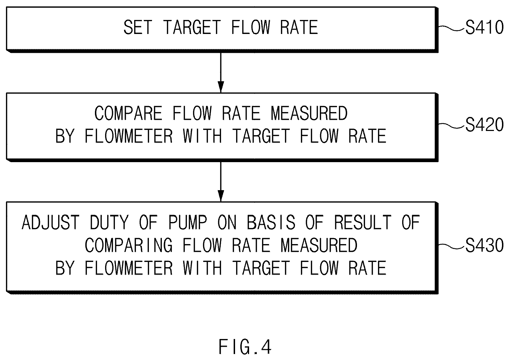

FIG. 4 is a flowchart for explaining a method of adjusting a flow rate of air sucked by the foreign substance detection device according to the embodiment disclosed in the present document.

With reference to FIG. 4, the step of adjusting, by the foreign substance detection device, the flow rate of the sucked air may include step S410 of setting the target flow rate, step S420 of comparing the flow rate measured by the flowmeter with the target flow rate, and step S430 of adjusting the duty of the pump on the basis of the result of comparing the flow rate measured by the flowmeter with the target flow rate.

In step S410, the controller 130 may set the target flow rate. The target flow rate may be set on the basis of a capacity of the inside of the foreign substance detection device 100, a cross-sectional area of the suction part 110, a rated flow rate of the sensor 120, and the like.

In step S420, the controller 130 may compare the flow rate, which is measured by the flowmeter 140, with the target flow rate.

In step S430, the controller 130 may adjust the duty of the pump 150 on the basis of the result of comparing the flow rate, which is measured by the flowmeter 140, with the target flow rate. Therefore, the controller 130 may maintain a constant flow rate condition during the operation of the foreign substance detection device 100.

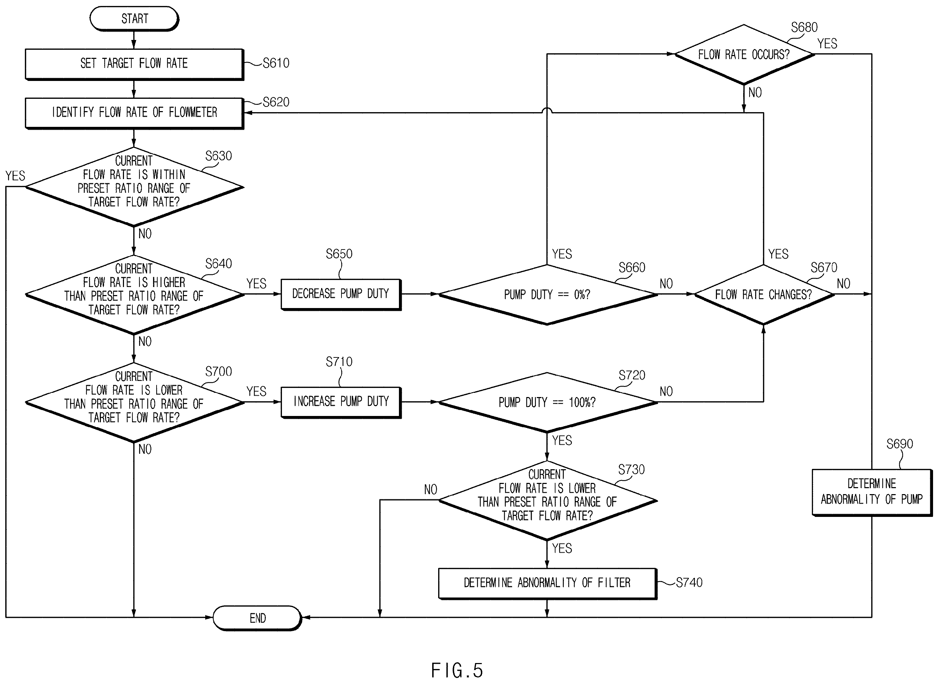

FIG. 5 is a flowchart illustrating a specific operating process of the foreign substance detection device according to the embodiment disclosed in the present document.

With reference to FIG. 5, the foreign substance detection device 100 may adjust the flow rate of the sucked air and determine whether the filter 160 and the pump 150 are abnormal.

In step S610, the controller 130 may set the target flow rate. The configuration in which the controller 130 sets the target flow rate may include a configuration in which the controller 130 receives information on the target flow rate from the user of the foreign substance detection device 100.

In step S620, the controller 130 may identify the flow rate of the flowmeter 140. The controller 130 may receive the measured flow rate information from the flowmeter 140.

In step S630, the controller 130 may identify whether the target flow rate and the current flow rate are consistent with each other. The current flow rate may refer to the flow rate identified by the flowmeter 140. The controller 130 may determine that the target flow rate and the current flow rate are consistent with each other in case that the current flow rate is within the preset ratio range of the target flow rate. The operation may end when the current flow rate is within the preset ratio range of the target flow rate (S630 - Yes). The process may go to step S640 in case that the current flow rate is not within the preset ratio range of the target flow rate (S630 - No).

In step S640, the controller 130 may determine whether the current flow rate exceeds the preset ratio range of the target flow rate. The process may go to step S650 in case that the current flow rate exceeds the preset ratio range of the target flow rate (S640 - Yes). The process may go to step S700 in case that the current flow rate does not exceed the preset ratio range of the target flow rate (S640 - No).

In step S650, the controller 130 may decrease the duty of the pump 150. More specifically, the controller 130 may decrease the duty of the pump 150 by a preset value.

In step S660, the controller 130 may identify whether the duty of the pump 150 is 0%. The process may go to step S680 in case that the duty of the pump 150 is 0% (S560 - Yes). The process may go to step S670 in case that the duty of the pump 150 is not 0% (S560 - No).

In step S670, the controller 130 may determine whether a change in flow rate has occurred. The controller 130 may determine the change in flow rate by receiving the flow rate information measured by the flowmeter 140. The process may go back to step S620 in case that the change in flow rate occurs (S670 - Yes). The process may go to step S690 in case that the change in flow rate does not occur (S670 - No).

In step S680, the controller 130 may determine that the flow rate has occurred. The process may go to step S690 in case that the flow rate occurs (S680 - Yes). The process may go back to step S620 in case that the flow rate does not occur (S680 - No).

In step S690, the controller 130 may determine that the pump 150 is abnormal.

In step S700, the controller 130 may determine whether the current flow rate is lower than the preset ratio range of the target flow rate. The process may go to step S710 in case that the current flow rate is lower than the preset ratio range of the target flow rate (S700 - Yes). The operation may end in case that the current flow rate is equal to or higher than the preset ratio range of the target flow rate (S700 - No).

In step S710, the controller 130 may increase the duty of the pump 150. More specifically, the controller 130 may increase the duty of the pump 150 by a preset value.

In step S720, the controller 130 may identify whether the duty of the pump 150 is 100%. The process may go to step S730 in case that the duty of the pump 150 is 1000% (S720 - Yes). The process may go to step S670 in case that the duty of the pump 150 is not 1000% (S720 - No).

In step S730, the controller 130 may determine whether the current flow rate is lower than the preset ratio range of the target flow rate. The process may go to step S740 in case that the current flow rate is lower than the preset ratio range of the target flow rate (S730 - Yes). The operation may end in case that the current flow rate is equal to or higher than the preset ratio range of the target flow rate (S730 - No).

In step S740, the controller 130 may determine that the filter 160 is abnormal.

FIG. 7 is a block diagram illustrating a hardware configuration of a computing system for performing a method of operating a battery management device according to the embodiment disclosed in the present document.

With reference to FIG. 7, a computing system 1000 according to the embodiment disclosed in the present document may include an MCU 1010, a memory 1020, an input/output I/F 1030, and a communication I/F 1040.

The MCU 1010 may be a processor configured to execute various types of programs (e.g., a battery cell SOC, an OCV collection program, a program for calculating a parameter of a battery cell, a program for creating an SOC-OCV assembly, a battery cell diagnosis program, and the like) stored in the memory 1020, process various types of information including the SOC, the OCV, the parameter, and the like of the battery cell by means of the programs, and perform the functions of the controller included in the battery management device illustrated in FIG. 2 described above.

The memory 1020 may store various types of programs such as the battery cell SOC, the OCV collection program, the program for calculating the parameter of the battery cell, the program for creating the SOC-OCV assembly, the battery cell diagnosis program, and the like. In addition, the memory 1020 may store various types of information including the SOC, the OCV, the parameter, and the like of the battery cell.

The memory 1020 may be provided as a plurality of memories 1020, as necessary. The memory 1020 may be a volatile memory or a non-volatile memory. A RAM, a DRAM, an SRAM, or the like may be used as the memory 1020 that is the volatile memory. A ROM, a PROM, an EAROM, an EPROM, an EEPROM, a flash memory, or the like may be used as the memory 1020 that is the non-volatile memory. The above-listed examples of the memory 1020 are merely illustrative, and the memory 1020 is not limited to this example.

The input/output I/F 1030 may provide an interface configured to connect an input device (not illustrated), such as a keyboard, mouse, or touch panel, and an output device, such as a display (not illustrated), to the MCU 1010 so that the input device, the output device, and the MCU may transmit and receive data.

The communication I/F 1040 may be various types of devices capable of transmitting and receiving various types of data to and from the server and supporting wired or wireless communication. For example, the battery management device may transfer and receive various types of information, which include the SOC, the OCV, the parameter, and the like of the battery cell, to and from an external server provided separately by means of the communication I/F 1040.

As described above, the computer program according to the embodiment disclosed in the present document may be implemented as a module recorded in the memory 1020 and processed by the MCU 1010 to perform the functions illustrated in FIG. 2, for example.

All the constituent elements, which constitute the embodiment disclosed in the present document described above, may be integrally coupled or operate by being combined, but the embodiments disclosed in the present document are not necessarily limited to the embodiment. That is, one or more of the constituent elements may be selectively combined and operated within the object of the embodiments disclosed in the present document.

In addition, unless explicitly described to the contrary, the words "comprise," "include," or "have" and variations such as "comprises," "comprising," "includes," "including," has," or "having," should be understood to imply the inclusion of stated elements but not the exclusion of any other elements. Unless otherwise defined, all terms including technical or scientific terms may have the same meaning as commonly understood by those skilled in the art to which the embodiments disclosed in the present document pertain. The terms such as those defined in a commonly used dictionary may be interpreted as having meanings consistent with meanings in the context of related technologies and may not be interpreted as ideal or excessively formal meanings unless explicitly defined in the present document.

The above description is simply given for illustratively describing the technical spirit disclosed in the present embodiment, and those skilled in the art to which the embodiments disclosed in the present document pertain will appreciate that various changes and modifications are possible without departing from the essential characteristics of the embodiments disclosed in the present document. Therefore, the embodiments disclosed in the present document are provided for illustrative purposes only but are not intended to limit the technical concept of the embodiments disclosed in the present document. The scope of the technical spirit disclosed in the present document is not limited by the embodiment. The protective scope of the technical spirit disclosed in the present document should be construed based on the appended claims, and all the technical spirit in the equivalent scope thereto should be construed as falling within the scope of the present document.

## Claims

1. A foreign substance detection device comprising:
a suction part configured to suck outside air at a target point;
a sensor configured to detect foreign substances contained in air sucked from the suction part;
a flowmeter configured to measure a flow rate of the air sucked from the suction part;
a pump configured to generate the flow rate; and
a controller configured to adjust a duty of the pump on the basis of the flow rate measured by the flowmeter and determine the amount of foreign substances at the target point on the basis of the foreign substances detected by the sensor.

2. The foreign substance detection device of claim 1, wherein the controller sets a target flow rate and adjusts the duty of the pump on the basis of a result of comparing the flow rate measured by the flowmeter with the target flow rate.

3. The foreign substance detection device of claim 2, wherein the controller decreases the duty by a preset value when the flow rate measured by the flowmeter is higher than a preset ratio range of the target flow rate, and
wherein the controller increases the duty by the preset value when the flow rate measured by the flowmeter is lower than the preset ratio range of the target flow rate.

4. The foreign substance detection device of claim 1, wherein the controller determines whether the pump is abnormal and whether a filter configured to remove the foreign substances is abnormal on the basis of the duty of the pump and the flow rate measured by the flowmeter.

5. The foreign substance detection device of claim 4, wherein the controller determines that the pump is abnormal when the flow rate is measured by the flowmeter in a state in which the duty of the pump 0% or when the flow rate measured by the flowmeter is not changed in a state in which the duty is changed.

6. The foreign substance detection device of claim 4, wherein the controller determines that the filter is abnormal when the flow rate measured by the flowmeter is lower than the target flow rate in a state in which the duty of the pump is 100%.

7. The foreign substance detection device of claim 1, wherein an angle and a length of the suction part are changed.

8. A method of operating a foreign substance detection device, the method comprising:
sucking outside air at a target point;
detecting foreign substances contained in the sucked air;
measuring a flow rate of the sucked air;
adjusting a duty of a pump, which is configured to generate the flow rate, on the basis of the measured flow rate; and
determining the amount of foreign substances at the target point on the basis of the detected foreign substances.

9. The method of claim 8, wherein the adjusting of the duty of the pump comprises:
setting a target flow rate;
comparing the measured flow rate with the target flow rate; and
adjusting the duty of the pump on the basis of a result of comparing the measured flow rate with the target flow rate.

10. The method of claim 8, further comprising:
determining whether the pump is abnormal and whether a filter configured to remove the foreign substances is abnormal on the basis of the duty of the pump and the measured flow rate.
